(19) **Europäisches Patentamt**
**European Patent Office**
**Office européen des brevets**

(11) **EP 4 674 517 A1**

(12) **EUROPEAN PATENT APPLICATION**
published in accordance with Art. 153(4) EPC

(43) Date of publication:
**07.01.2026 Bulletin 2026/02**

(21) Application number: **24763958.6**

(22) Date of filing: **28.02.2024**

(51) International Patent Classification (IPC):
*B01D 71/26* $^{(2006.01)}$    *A61M 1/18* $^{(2006.01)}$
*B01D 63/02* $^{(2006.01)}$    *B01D 69/00* $^{(2006.01)}$
*B01D 69/02* $^{(2006.01)}$    *B01D 69/08* $^{(2006.01)}$
*B01D 69/12* $^{(2006.01)}$    *B01D 71/76* $^{(2006.01)}$

(52) Cooperative Patent Classification (CPC):
**B01D 63/02; B01D 69/00; B01D 69/02;**
**B01D 69/08; B01D 69/12; B01D 71/26; B01D 71/76**

(86) International application number:
**PCT/JP2024/007213**

(87) International publication number:
**WO 2024/181479 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.02.2023 JP 2023029465**

(71) Applicant: **Toray Industries, Inc.**
**Tokyo 103-8666 (JP)**

(72) Inventors:
• **IWAKAWA Sari**
**Otsu-shi, Shiga 520-8558 (JP)**
• **FUJITA Masaki**
**Otsu-shi, Shiga 520-8558 (JP)**

(74) Representative: **Kador & Partner Part mbB**
**Corneliusstraße 15**
**80469 München (DE)**

(54) **HOLLOW FIBER MEMBRANE, METHOD FOR MANUFACTURING HOLLOW FIBER MEMBRANE, AND HOLLOW FIBER MEMBRANE MODULE**

(57)    [Problem] An object of the present invention is to provide: a hollow fiber membrane having biocompatibility stable for a long time, and containing, as a main component, polymethylpentene having excellent gas permeability in the membrane; and a method of producing the hollow fiber membrane.

[Solution] A hollow fiber membrane including polymethylpentene as a main component, and having a dense surface having a hydrophilic polymer immobilized therein, wherein the thickness of a layer containing the hydrophilic polymer is 1.0 nm or more and 25.0 nm or less.

EP 4 674 517 A1

**Description**

Technical Field

The present invention relates to a hollow fiber membrane, a method of producing the hollow fiber membrane, and a hollow fiber membrane module.

Background Art

A membrane oxygenator produced using a porous membrane is commonly and widely used as an extracorporeal circulation machine for a cardiotomy for a heart disease, an artificial heart-lung machine for circulatory support or an extracorporeal membrane oxygenation (hereinafter referred to as ECMO) machine that is a substitute machine for a respiratory function. A membrane oxygenator is produced mainly using a hollow fiber membrane. Via the hollow fiber membrane, gas exchange is performed in blood. Examples of a method of perfusing blood into an oxygenator include: an internal perfusion method in which blood is made to flow through the inside of hollow fiber membranes. and gas is made to flow through the outside of the hollow fiber membranes; and an external perfusion method in which blood is made to flow through the outside of hollow fiber membranes. and gas is made to flow through the inside of the hollow fiber membranes.

A hollow fiber membrane oxygenator involves contact between blood and the inner surface or outer surface of the hollow fiber membrane. Thus, the inner surface or outer surface of the hollow fiber membrane. brought into contact with blood, influences the adhesion or activation of platelets. In particular, an external perfusion method in which the outer surface of a hollow fiber membrane comes into contact with blood causes the flow of the blood to be turbulence, and thus, is prone to influence the adhesion or activation of platelets.

If a medical separation membrane that comes into contact with a bodily fluid or blood allows protein or platelets to adhere to the membrane, the adhesion will cause a decrease in the performance of the separation membrane, or cause a biological reaction, thus resulting in a serious problem. In the case of an oxygenator in particular, the adhesion of protein or platelets to the surface of the hollow fiber membrane or the adhesion of protein to the surface of the hollow fiber membrane and the insides of the pores of the membrane causes the surface and inside of the hollow fiber membrane to lose hydrophobicity and be hydrophilized, thereby causing, for example, leakage (plasma leak) in which a blood plasma component exudes in the cross-sectional direction of the hollow fiber membrane. Thus, the membrane results in having a decreased area usable for gas exchange, decreasing in the gas exchange capability. Accordingly, the hollow fiber membrane is required to have biocompatibility for inhibiting the adhesion of protein or platelet, and various studies are being made.

As a hollow fiber membrane for an oxygenator, a polyolefin hollow fiber membrane having gas permeability and hydrophobicity is suitably used. Among others, a hollow fiber membrane containing polymethylpentene as a main component has a dense structure on the outer surface side that is a blood-contacting face. Thus, the hollow fiber membrane is often utilized for the purpose of preventing plasma leak.

A coating method is disclosed (Patent Literature 1), which allows the surface to have a polymer containing alkoxyalkyl(meth)acrylamide as a main constituent, in order to give biocompatibility to such a polyolefin. In addition, a method of coating the surface with polymethoxyethyl acrylate (Patent Literature 2) and a method of coating the surface with a copolymer of alkoxyalkyl methacrylate and hydroxyalkyl methacrylate (Patent Literature 3) are disclosed. In addition, a method in which heparin is used is disclosed (Patent Literature 4) as a coating method for enhancing antithrombogenicity for an oxygenator.

A technique in which a hydrophilic polymer such as vinylpyrrolidone is added to inhibit adhesion of platelets or protein safely and simply is utilized not only for a hollow fiber membrane for an oxygenator but also for others.

For example. a method is disclosed in which polyvinylpyrrolidone (Patent Literature 5) or a copolymer of polyvinylpyrrolidone and vinyl acetate (Patent Literature 6) is added to a membrane through radiation graft polymerization.

Prior Art References

Patent Literature

Patent Literature 1: JP 2004-357826 A

Patent Literature 2: JP H11-114056 A

Patent Literature 3: JP 2020-141901 A

Patent Literature 4: JP 2020-127705 A

Patent Literature 5: JP H03-16626 A

Patent Literature 6: JP 2011-78974 A

Non-Patent Literature

Non-Patent Literature 1: M. Niinomi, "Metals for Biomedical Devices", 2nd Edition, Elsevier, 2019, p. 405

Summary of Invention

Problem Which the Invention Tries to Solve

In the method described in Patent Literature 1, the coating layer has a large thickness of 0.1 μm to 1 mm, and accordingly causes the original substance permeability of the membrane to decrease. Thus, the method is not preferable. In addition, the coating layer. which is thick, causes the hydrophilicity of the membrane to increase. raising concern that a long-time contact between the coating layer and blood will cause plasma leak. In addition, in the method described in Patent Literature 2, polymethoxyethyl acrylate exhibits excellent biocompatibility, but an organic solvent, such as methanol which is a deleterious substance, needs to be used as a solvent for coating, thus raising concern that contact between the membrane and the organic solvent causes the microstructure of the membrane to be deformed. These coating methods may require structure control for maintaining the performance of the membrane, each time the condition is changed.

Patent Literature 3 discloses a method in which a polypropylene base material is coated with a copolymer dissolved in a mixed solvent of water and alcohol (hereinafter, the method is referred to as a "cast method"). In general, however, in cases where a base material is coated with a polymer using the cast method, the adhesiveness and durability of the coating component varies depending on whether the surface of the base material has a microstructure that is, for example, uneven, and whether the surface of the base material has hydrophilicity or hydrophobicity. Thus, the method is unsuitable for polymethylpentene that affords a dense surface having less unevenness. and is strongly hydrophobic. In addition, in the cast method, the molecular weight of a polymer usable for coating is limited because a polymer having a small molecular weight has low adhesiveness to a base material, and tends to be more difficult to use for coating.

The method described in Patent Literature 4 has a problem of development of allergy because heparin is a biologically derived material. Furthermore, heparin itself is consumed along with the inhibition of coagulation of blood, thus, causing a problem of difficulty in maintaining the blood-coagulation-suppressing effect during long-time use.

In the method described in Patent Literature 5 or Patent Literature 6, a hydrophilic polymer is present in the whole membrane. Thus, in cases where the hollow fiber membrane is used as a gas-exchange membrane, the hydrophilic polymer contributes to the infiltration of a blood plasma component into the pores in the membrane, raising concern about propelling the occurrence of plasma leak. These effects have been verified only with a membrane for protein permeation applications in the whole of which membrane a hydrophilic polymer is present and have not been verified with a hollow fiber membrane for an oxygenator in which the membrane is intended for gas exchange, and needs to retain the hydrophobicity of the membrane.

Furthermore, it is known that polyolefin, particularly polymethylpentene, generally has low radiation resistance (Non-Patent Literature 1). Accordingly, there is concern that polymethylpentene irradiated will vary the separation performance and mechanical characteristics of the membrane. Thus, no study has been made to use such a membrane for a gas-exchange membrane and a separation membrane, such as for an oxygenator.

As described above, the method including adding a hydrophilic polymer containing vinylpyrrolidone is simple and highly effective, but on the other hand. the membrane is hydrophilized in the cross-sectional direction thereof, depending on how much and/or how the polymer is added. Thus, use of the method for a hollow fiber membrane for an oxygenator has not been achieved.

In view of this, an object of the present invention is to provide: a hollow fiber membrane having biocompatibility stable for a long time, and containing, as a main component, polymethylpentene having excellent gas permeability in the membrane; and a method of producing the hollow fiber membrane.

Solution to Problem

The present inventors have made studies vigorously to solve the above-described problems. As a result, the constitution of the present invention for solving the above-described problems is as follows.

(1) A hollow fiber membrane comprising polymethylpentene as a main component, and having a dense surface having a hydrophilic polymer immobilized therein, wherein the thickness of a layer containing the hydrophilic polymer is 1.0 nm or more and 25.0 nm or less.

(2) The hollow fiber membrane according to (1), wherein the hydration energy density of the hydrophilic polymer is 167 kJ·mol$^{-1}$·nm$^{-3}$ or more and 213 kJ ·mol-l·nm$^{-3}$ or less.

(3) The hollow fiber membrane according to (1) or (2), comprising a nitrogen atom in an amount of 0.0010 mass% or more and 0.0100 mass% or less.

(4) The hollow fiber membrane according to any one of (1) to (3), wherein the hydrophilic polymer comprises a hydrophilic unit selected from the group consisting of a vinylpyrrolidone unit, acrylamide derivative unit, methacrylamide derivative unit, N-vinylacetamide derivative unit, and vinylcaprolactam unit. wherein the amount of the hydrophilic unit is 0.010 mass% or more and 0.080 mass% or less.

(5) The hollow fiber membrane according to any one of (1) to (4). wherein the contact angle of water on the dense surface is 70° or more and 95° or less.

(6) The hollow fiber membrane according to any one of (1) to (5), wherein the hydrophilic polymer comprises a hydrophobic unit selected from the group consisting of a vinyl carboxylate unit, alkyl acrylate unit, and alkyl methacrylate unit.

(7) The hollow fiber membrane according to (6), wherein the number of carbon atoms of a side-chain end of the hydrophobic unit is 1 or more and 9 or less.

(8) A method of producing the hollow fiber membrane according to any one of (1) to (7), comprising the following Step 1:

Step 1: a step of irradiating, with a radiation, the hollow fiber membrane brought into contact with a hydrophilic solution.

(9) The method of producing a hollow fiber membrane according to (8), wherein the hydrophilic solution is brought into contact with only one surface of the hollow fiber membrane.

(10) A hollow fiber membrane module in which the hollow fiber membrane according to any one of (1) to (6) is packed.

(11) The hollow fiber membrane module according to (10), which is an oxygenator.

In addition, the constitution of the present invention for solving the above-described problems is as follows.

(12) A hollow fiber membrane comprising polymethylpentene as a main component, and having a dense surface having a hydrophilic polymer immobilized thereon, wherein the thickness of a layer containing the hydrophilic polymer is 1.0 nm or more and 25.0 nm or less.

(13) The hollow fiber membrane according to (12), wherein the hydration energy density of the hydrophilic polymer is 167 kJ·mol$^{-1}$·nm$^{-3}$ or more and 213 kJ·mol$^{-1}$·nm$^{-3}$ or less.

(14) The hollow fiber membrane according to (12) or (13), comprising a nitrogen atom in an amount of 0.0015 mass% or more and 0.0100 mass% or less.

(15) The hollow fiber membrane according to any one of (12) to (14), wherein the hydrophilic polymer comprises a vinylpyrrolidone unit, wherein the amount of the vinylpyrrolidone unit is 0.010 mass% or more and 0.080 mass% or less.

(16) The hollow fiber membrane according to any one of (12) to (15), wherein the contact angle of water on the dense surface is 80° or more and 95° or less.

(17) The hollow fiber membrane according to any one of (12) to (16), wherein the hydrophilic polymer comprises a vinyl carboxylate unit.

(18) The hollow fiber membrane according to (17), wherein the number of carbon atoms of a side-chain end of the vinyl carboxylate unit is 1 to 9.

(19) A method of producing the hollow fiber membrane according to any one of (12) to (18), comprising the following Step 1:

Step 1: a step of irradiating, with a radiation, the hollow fiber membrane brought into contact with a hydrophilic solution.

(20) The method of producing a hollow fiber membrane according to (19), wherein the hydrophilic solution is brought into contact with only one surface of the hollow fiber membrane.

(21) A hollow fiber membrane module in which the hollow fiber membrane according to any one of (12) to (18) is packed.

(22) The hollow fiber membrane module according to (21), which is an oxygenator.

Effects of the Invention

A hollow fiber membrane and a hollow fiber membrane module according to the present invention allows less adhesion of an organismic component, such as protein or platelets. In addition, the hollow fiber membrane and the hollow fiber membrane module have excellent gas permeability. and thus, can be utilized as an oxygenator.

Brief Description of Drawings

FIG. 1 is the photographs showing the states of adhesion of the platelets in Examples 1 to 10.

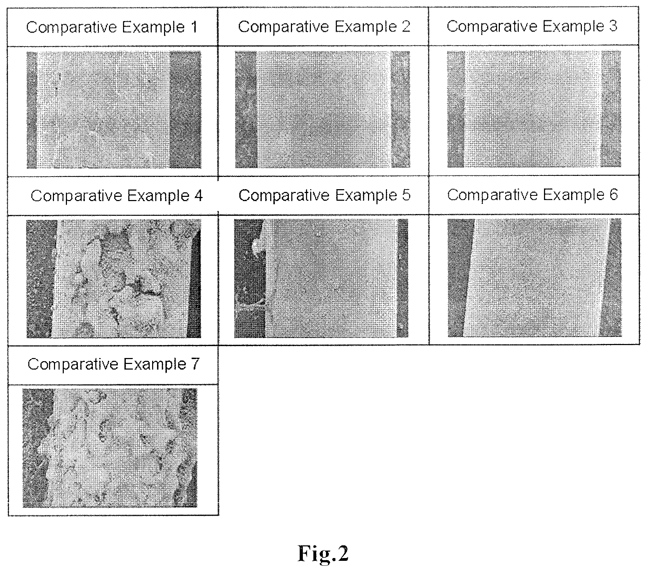

FIG. 2 is the photographs showing the states of adhesion of the platelets in Comparative Examples 1 to 7.
FIG. 3 is the photographs showing the states of adhesion of the platelets in Examples 11 to 23.

Mode for Carrying Out the Invention

The present invention is a hollow fiber membrane comprising polymethylpentene (hereinafter referred to as "PMP") as a main component, and having a dense surface having a hydrophilic polymer immobilized therein, wherein the thickness of a layer containing the hydrophilic polymer is 1.0 nm or more and 25.0 nm or less.

The "main component" means a material constituting 50 mass% or more of the whole of the materials constituting the hollow fiber membrane. From the viewpoints of forming a dense surface structure and maintaining the mechanical strength, PMP preferably accounts for 90 mass% or more of the whole hollow fiber membrane, more preferably 95 mass% or more.

For a hollow fiber membrane according to the present invention, it is preferable that the distribution of the pores in the hollow fiber membrane is ununiform in the cross-sectional direction of the membrane, and that the membrane has an asymmetric structure in which the inner and outer surfaces have different structures. Having the dense surface of the hollow fiber membrane as a contacting face for a blood component makes it possible to achieve both gas permeability and inhibition of plasma leak.

The "dense surface" means a surface of the hollow fiber membrane having an asymmetric structure, in which the surface has the smaller pore size. For example, in cases where the pore size of the outer surface is smaller than the pore size of the inner surface, the outer surface is the dense surface.

A hollow fiber membrane according to the present invention contains a hydrophobic PMP as a main component, and thus, the inner surface and outer surface of the hollow fiber membrane are hydrophobic. Accordingly, the surfaces, if used directly, tend to allow adhesion of an organismic component, such as protein or platelets. Thus, the hydrophilic polymer needs to be immobilized in the dense surface that comes into contact with blood.

Being "immobilized" means a state of being not easily removable even by washing with a solvent in which the hydrophilic polymer can be dissolved. Examples of the state of being immobilized include a state in which the hydrophilic polymer and PMP are mutually dissolved in each other to have the molecular chains entangled with each other or bound with each other via a covalent bond or an ionic bond. Among these, a state of being bound via a covalent bond is preferable because of low concern about elution during contact with blood.

Immobilizing a hydrophilic polymer on the hollow fiber membrane makes it possible to suppress adhesion of an organismic component. On the other hand, in cases where the amount of immobilization of the hydrophilic polymer is large, the whole membrane becomes hydrophilic, raising concern of generation of plasma leak. Furthermore, there is a possibility that the hollow fiber membrane is decreased in the gas permeability. becoming insufficient in performance when used as an oxygenator.

The term "Biocompatibility" means a property by which adhesion of platelets and/or protein is suppressed, and by which thrombus formation is made less prone to occur. Giving biocompatibility to that surface of the hollow fiber membrane which comes into contact with blood prevents a thrombus from being formed on the surface of the membrane, prevents the membrane from decreasing in the area contributive to gas exchange, and thus, enables the membrane to maintain the gas-permeating performance. In addition, it is made possible to suppress adhesion of protein inside the pores, and to prevent plasma leak accompanying hydrophilization of the hollow fiber membrane.

In view of this. the present inventors have advanced a study vigorously. and as a result. have discovered that having 1.0 nm or more and 25.0 nm or less as the thickness of the hydrophilic-polymer-containing layer in the dense surface of the hollow fiber membrane enables the membrane to achieve both biocompatibility and gas permeability.

The term "hydrophilic-polymer-containing layer" means the etching depth for which, in gas cluster ion beam time-of-flight secondary-ion mass spectrometry (hereinafter referred to as "GCIB-TOF-SIMS"), the peak of the secondary ion derived from a hydrophilic polymer is detected when the membrane is etched in the thickness direction from the surface of the membrane. That is, the layer is different from the thickness of a hydrophilic polymer layer present like a coating film in the surface of the hollow fiber membrane, and means a layer containing a hydrophilic polymer that has permeated inside the hollow fiber membrane from the pores of the dense surface. and been immobilized. Specifically, the etching depth can be measured using the method described in "Thickness of hydrophilic-polymer-containing layer" below.

It is generally considered that the adhesion of protein or platelets to the surface of a material is caused as follows: the higher order structure of protein changes, and thus causes the hydrophobic site to be exposed on the surface, and undergo hydrophobic interaction with the surface of the material, causing the adhesion. In addition, around a cell, such as a platelet, and protein and on the surface of the material, water bound by hydrogen bonding, i.e., what is called bound water, is present. Accordingly, it is said that the hydrophilicity on the surface of the material, when strong, causes the bound water around the protein to be also bound, not making it possible to sufficiently inhibit the adhesion of protein. That is, it can be presumed that, to suppress the adhesion of a cell, such as a platelet, and protein, it is important to suitably adjust a balance between the hydrophobicity and hydrophilicity of the surface material.

As described above, giving biocompatibility and maintaining the gas-permeating performance can be achieved by providing a hydrophilic-polymer-containing layer having a given thickness in the surface of the hollow fiber membrane so that the balance between hydrophilicity and hydrophobicity can be made suitable, wherein the surface comes into contact with an organismic component, such as blood.

From the viewpoint of giving biocompatibility, the thickness of the hydrophilic-polymer-containing layer is 1.0 nm or more, preferably 3.0 nm or more. On the other hand, in cases where the hydrophilic polymer is present inside the pores of the hollow fiber membrane, an organismic component permeates into the pores more easily, causing an increase in the amount of adhesion of protein inside the pores, and making plasma leak more likely. In addition, a hydrophilic-polymer-containing layer having a larger thickness results in having larger permeation resistance, and being decreased in gas permeability. Accordingly, from the viewpoint of maintaining plasma-leak resistance and the gas permeability of the hollow fiber membrane, the thickness of the hydrophilic-polymer-containing layer is 25.0 nm or less. preferably 15.0 nm or less, more preferably 5.0 nm or less.

The "hydrophilic polymer" means a polymer having a water solubility of 0.00001 mass% or more at room temperature (20°C), and having a hydrophilic unit.

The term "hydrophilic unit" means a repeating unit of a polymer composed of a hydrophilic unit alone, the water solubility of which polymer is 1 mass% or more at room temperature (20°C).

The thickness of the hydrophilic-polymer-containing layer can be controlled by adjusting the hydrophilicity and hydrophobicity of the hydrophilic polymer, or, for example, in accordance with the concentration of the hydrophilic polymer in a hydrophilic solution used for irradiation.

The hydration energy density of the hydrophilic polymer to be immobilized in a hollow fiber membrane according to the present invention is preferably 167 $kJ \cdot mol^{-1} \cdot nm^{-3}$ or more and 213 $kJ - mol^{-1} \cdot nm^{-3}$ or less. Having 167 $kJ \, mol^{-1} \cdot nm^{-3}$ or more and 213 $kJ \cdot mol^{-1} \cdot nm^{-3}$ or less as the hydration energy density of the hydrophilic polymer affords biocompatibility sufficiently, enables the hollow fiber membrane to retain the hydrophobicity, makes plasma leak less prone to occur, and thus, is preferable. To make the hydrophilicity and the hydrophobicity suitable, the hydration energy density is preferably 170 $kJ \cdot mol^{-1} \cdot nm^{-3}$ or more and 190 $kJ \cdot mol^{1} \cdot nm^{-3}$ or less, more preferably 175 $kJ \cdot mol^{-1} \cdot nm^{-3}$ or more and 190 $kJ \cdot mol^{-1} \cdot nm^{-3}$ or less.

In addition, in cases where a hydrophilic polymer is added to polymethylpentene, which is hydrophobic, through radiation graft polymerization, allowing the polymer itself to have a given hydrophobicity increases the equilibrium constant of adsorption of the polymer into the hollow fiber membrane. making it easier for the hydrophilic polymer to be added to the hollow fiber membrane. Thus, the hydration energy density of the hydrophilic polymer is preferably 167 $kJ \cdot mol^{-1} \cdot nm^{-3}$ or more and 213 $kJ \cdot mol^{-1} \cdot nm^{-3}$ or less.

The hydration energy density of the hydrophilic polymer is calculated on the basis of the following Formula (1).
[Math. 1]

$$\text{Hydration energy density of copolymer } (kJ \cdot mol^{-1} \cdot nm^{-3}) =$$

$$\sum_{i=1}^{N} \{(\text{Molar fraction of monomer unit } i) \times (\text{Hydration energy of monomer unit } i)\} /$$

$$\sum_{i=1}^{N} \{(\text{Molar fraction of monomer unit } i) \times (\text{Volume of monomer unit } i)\} \dots$$

Formula (1)

[in Formula (1), the hydration energy of the monomer unit i is the absolute value of a value obtained by subtracting the energy of the monomer unit i in vacuum from the energy of the monomer unit i in water; N represents the total number of monomer species constituting the copolymer; and i represents an integer of 1 or less and N or less.]

The term "hydration energy" means an energy variation obtained by a system when a solute is put into an aqueous solution. A unit to be used for the hydration energy is, for example, $J \cdot mol^{-1}$.

The term "hydration energy of a monomer unit" means the absolute value of a value obtained by subtracting the energy of the monomer unit in vacuum from the energy of the monomer unit in water.

The term "hydration energy density" means hydration energy per unit volume. The unit to be used for hydration energy density is $kJ \cdot mol^{-1} \cdot nm^{-3}$ on the basis of the unit of hydration energy.

The energy of a monomer unit in vacuum and the energy of the monomer unit in water can be calculated using the method described in "Hydration energy density of hydrophilic polymer" below.

The hydration energy density of the hydrophilic polymer can be controlled in accordance with a combination of monomers. for example, hydrophilic units constituting the hydrophilic polymer.

The nitrogen atom content of the hollow fiber membrane according to the present invention is preferably 0.0010 mass% or more and 0.0100 mass% or less, more preferably 0.0015 mass% or more and 0.0100 mass% or less.

The term "nitrogen atom content" means the content (mass%) of nitrogen atoms in 100 mass% of the hollow fiber membrane. Among others, the nitrogen atoms are preferably derived from a hydrophilic polymer.

As the amount of nitrogen atoms derived from a hydrophilic polymer, 0.0010 mass% or more affords sufficient biocompatibility, and 0.0015 mass% or more affords more preferable biocompatibility. On the other hand, from the viewpoints of retaining the hydrophobicity of the hollow fiber membrane, suppressing formation of gel generated by the cross-linking of the hydrophilic polymer itself, and enabling the hollow fiber membrane to maintain the gas permeability, the nitrogen atom content is preferably 0.0100 mass% or less, more preferably 0.0070 mass% or less, still more preferably 0.0060 mass% or less, still more preferably 0.0050 mass% or less.

The nitrogen atom content of the hollow fiber membrane can be measured and determined by the oxidative decomposition-chemiluminescence method, for example, using a trace nitrogen analyzer (model ND-100, Mitsubishi Chemical Corporation).

The nitrogen content of the hollow fiber membrane can be controlled by adjusting the hydrophilicity and hydrophobicity of the hydrophilic polymer, or by adjusting the concentration of the hydrophilic polymer in a hydrophilic solution used for irradiation.

In cases where a hollow fiber membrane according to the present invention contains a nitrogen atom derived from the hydrophilic polymer, the nitrogen atom is preferably derived from an amide bond other than a secondary amide bond, more preferably derived from a polymer having a heterocyclic compound containing an amine, a primary or tertiary amide bond, or a nitrogen atom.

Specific examples of the hydrophilic unit contained in the hydrophilic polymer that satisfies the above-described conditions include: amines, such as vinylamine, allylamine, lysine. and diallylmethyl ammonium chloride; units having primary or tertiary amide bonds, such as methacrylamide, ethyl carbamate, $N,N$-dimethylmethacrylamide, $N$-vinylacetamide, N-isopylacrylamide, and vinylpyrrolidone; and units having a heterocyclic compound containing a nitrogen atom, such as 2-vinylpyridine.

What functional group the nitrogen atom contained is derived from can be verified by identifying the secondary ion species containing the nitrogen atom through time-of-flight secondary-ion mass spectrometry (hereinafter referred to as "TOF-SIMS measurement"), or by extracting a hydrophilic polymer with an organic solvent, such as ethanol, butanol, or hexane, and making a measurement through proton nuclear magnetic resonance spectroscopy (hereinafter referred to as "$^1$H-NMR measurement"). Furthermore, in cases where the hydrophilic polymer contains an amide group, the ratio of the amide bond can be calculated from the ratio of the peak area derived from the amide bond, using a $^1$H-NMR measurement.

In addition. in cases where a functional group containing a nitrogen atom is present in a side chain in the hydrophilic polymer, the molecular weight of the side chain is preferably smaller so as to enable biocompatibility to be given, and so as to make it easier for the gas permeability to be maintained, in a state where the hydrophilic-polymer-containing layer has a smaller thickness.

The hydrophilic polymer immobilized in the hollow fiber membrane according to the present invention preferably comprises a hydrophilic unit selected from the group consisting of a vinylpyrrolidone unit, acrylamide derivative unit, methacrylamide derivative unit, N-vinylacetamide derivative unit, and vinylcaprolactam unit. The hydrophilic unit content is preferably 0.0100 mass% or more and 0.0800 mass% or less.

Among these, a vinylpyrrolidone unit, acrylamide derivative unit, or $N$-$N$-vinylacetamide derivative vinyl carboxylate unit is more preferable, and a vinylpyrrolidone unit is still more preferable, from the viewpoint of having smaller modifying action and activating action on an organismic component.

The acrylamide derivative unit means a unit having the acrylamide structure ($CH_2$=CH-CO-NH-). Examples of the acrylamide derivative unit include an acrylamide unit, $N$-methylacrylamide unit, N-isopropylacrylamide unit, N-tert-butylacrylamide unit, and N-phenylacrylamide unit.

The methacrylamide derivative unit means a unit having the methacrylamide structure ($CH_2$=C($CH_3$)-CO-NH-). Examples of the methacrylamide derivative unit include methacrylamide unit, N-isopropylmethacrylamide unit, and $N$-phenylmethacrylamide unit.

The N-vinylacetamide derivative unit means a unit having the vinylacetamide structure ($CH_2$=CH-NH-CO-). Examples of the N-vinylacetamide derivative unit include N-vinylacetamide unit and $N$-methyl-$N$-vinylacetamide unit.

The "hydrophilic unit content" means the content (mass%) of the hydrophilic unit contained in 100 mass% of the hollow fiber membrane. For example, in cases where the hydrophilic unit is vinylpyrrolidone unit, the hydrophilic unit content means the content (mass%) of the vinylpyrrolidone unit contained in 100 mass% of the hollow fiber membrane.

From the viewpoint of obtaining sufficient biocompatibility, the hydrophilic unit content is more preferably 0.010 mass% or more. On the other hand, from the viewpoints of maintaining the hydrophobicity of the hollow fiber membrane, and suppressing plasma leak, or from the viewpoint of suppressing the decrease in the gas permeability of the hollow fiber membrane by the cross-linking of the hydrophilic polymer itself, the hydrophilic unit content is more preferably 0.050 mass% or less, still more preferably 0.035 mass% or less.

Among the contents of the hydrophilic units, for example, the vinylpyrrolidone unit content can be calculated using the method described in "Vinylpyrrolidone unit content" below.

The static contact angle of water on the dense surface of the hollow fiber membrane according to the present invention is preferably 70° or more and 95° or less. Having 70° or more, more preferably 75° or more, still more preferably 80° or more, as the static contact angle of water on the dense surface of the hollow fiber membrane makes it possible to maintain the hydrophobicity of the hollow fiber membrane. and to suppress the occurrence of plasma leak. On the other hand, from the viewpoint of obtaining sufficient biocompatibility, the static contact angle of water is preferably 95° or less, more preferably 90° or less.

The static contact angle of water on the dense surface of the hollow fiber membrane can be measured by the sessile drop method, for example, using an automatic microscopic contact angle meter (MCA-4, Kyowa Interface Science Co.. Ltd.).

In addition, the hydrophilic polymer of the hollow fiber membrane according to the present invention preferably has a hydrophobic unit selected from the group consisting of a vinyl carboxylate unit, alkyl acrylate unit, and alkyl methacrylate unit. The hydrophobic unit is hydrophobic. Having a hydrophobic unit present in the hydrophilic polymer makes it possible to optimize the biocompatibility-giving effect of the hydrophilic polymer. Among these, a vinyl carboxylate unit is more preferable, from the viewpoint of having a smaller stimulating action and activating action on an organismic component, such as a blood corpuscle.

The vinyl carboxylate unit means a repeating unit represented by "$-CH(OCO-R)-CH_2-$" (R is a hydrocarbon group). Examples of the vinyl carboxylate unit include vinyl propionate unit, vinyl butanoate unit, vinyl pivalate unit, vinyl pentanoate unit, vinyl octanoate unit, vinyl 2-ethylhexanoate unit. and vinyl stearate unit.

Examples of the alkyl acrylate unit include ethyl acrylate unit, propyl acrylate unit, butyl acrylate unit, isobutyl acrylate unit, *tert*-butyl acrylate unit, octyl acrylate unit, and hexadecyl acrylate unit.

Examples of the alkyl methacrylate unit include ethyl methacrylate unit, propyl methacrylate unit, butyl methacrylate unit, isobutyl methacrylate unit, *tert*-butyl methacrylate unit, tridecyl methacrylate unit, 1-butene unit, and l-nonene unit.

In general, the larger the number of carbon atoms of the side-chain end R of the hydrophobic unit, the higher the hydrophobicity. Thus, to keep the hydrophobicity of the hollow fiber membrane having a hydrophilic polymer added thereto, 1 or more is suitably used as the number of carbon atoms of the hydrophobic side-chain end R.

Furthermore, the number of carbon atoms of the side-chain end of the hydrophobic unit is preferably 1 or more and 9 or less. In cases where the hydrophobicity of the hydrophilic polymer itself is strong, the water solubility of the hydrophilic polymer is decreased, and thus, the number of carbon atoms of the side-chain end R of the hydrophobic unit is preferably smaller, more preferably 9 or less.

Examples of the vinyl carboxylate unit in which the number of carbon atoms of the side-chain end R of the hydrophobic unit is 1 or more and 9 or less include vinyl acetate ($C_1$), vinyl propionate ($C_2$), vinyl butanoate ($C_3$), vinyl pentanoate ($C_4$), vinyl hexanoate ($C_5$). vinyl heptanoate ($C_6$), vinyl nonanoate ($C_8$), and vinyl decanoate ($C_9$).

In addition, the side-chain end R of the hydrophobic unit may contain a branched structure, such as an isopropyl group or a tertiary butyl group. Examples thereof include vinyl pivalate ($C_4$) or vinyl 2-ethylhexanoate ($C_7$). In addition, allowing the hydrophobic unit as a side chain of the hydrophilic polymer to have a smaller molecular weight makes it possible to give biocompatibility, and makes it easier to maintain the gas permeability, in a state where the hydrophilic-polymer-containing layer has a smaller thickness. In cases where the hydrophobic unit is a vinyl carboxylate unit, the number of carbon atoms of the side-chain end R is more preferably 1 or more and 7 or less, still more preferably 1 or more and 5 or less. For example, the vinyl carboxylate unit is particularly preferably a $C_1$ vinyl acetate unit or $C_2$ vinyl propionate.

The hydrophilic polymer in the present invention preferably has a hydrophilic unit selected from the group consisting of vinylpyrrolidone unit, acrylamide derivative unit, and N-N-vinylacetamide derivative vinyl carboxylate unit, and has a hydrophobic unit selected from the group consisting of a vinyl carboxylate unit, alkyl acrylate unit, and alkyl methacrylate unit. In addition. the molar fraction of each unit in a copolymer of the hydrophilic unit and the hydrophobic unit varies depending on the structure of the hydrophobic unit, and is preferably 0.1 or more and 0.8 or less, in general. The hydration energy density calculated on the basis of the above-described Formula (1) preferably satisfies 167 $kJ \cdot mol^{-1} \cdot nm^{-3}$ or more and 213 $kJ \cdot mol^{-1} \cdot nm^{-3}$ or less.

In cases where the hydrophobic unit is a vinyl carboxylate unit, the larger the number of carbon atoms is, the stronger the hydrophobicity is. Thus, in cases where the number of carbon atoms of a vinyl carboxylate is large, the molar fraction of the vinyl carboxylate unit in the hydrophilic polymer is preferably lower. In particular, in cases where the vinyl carboxylate unit is a vinyl acetate unit. vinyl propionate unit, vinyl butanoate unit, or vinyl pivalate unit, the molar fraction in the hydrophilic polymer is preferably 0.2 or more and 0.8 or less. more preferably 0.35 or more and 0.65 or less. In addition, from the viewpoint of water solubility, the molar fraction of the vinyl carboxylate unit is still more preferably 0.35 or more and 0.60 or less. Furthermore, considering handleability. the molar fraction is particularly preferably 0.35 or more and 0.50 or less.

In cases where the hydrophobic unit is an alkyl acrylate unit, the larger the number of carbon atoms is, the stronger the hydrophobicity is. Thus, in cases where the number of carbon atoms of a vinyl acrylate is large, the molar fraction of the vinyl acrylate unit in the hydrophilic polymer is preferably lower. In particular, in cases where the alkyl acrylate unit is ethyl acrylate, the molar fraction in the hydrophilic polymer is preferably 0.2 or more and 0.8 or less, more preferably 0.35 or more and 0.65 or less. In addition, from the viewpoint of water solubility, the molar fraction of the vinyl acrylate unit is still more preferably 0.40 or more and 0.60 or less. Furthermore, considering handleability, the molar fraction is particularly preferably 0.45 or more and 0.55 or less.

In cases where a copolymer having a hydrophilic unit selected from the group consisting of a vinylpyrrolidone unit, acrylamide derivative unit, and *N-N*-vinylacetamide derivative vinyl carboxylate unit, and having a hydrophobic unit selected from the group consisting of a vinyl carboxylate unit and an alkyl acrylate is used, an alternating copolymer or a random copolymer can be used. In these cases, the vinylpyrrolidone unit and the vinyl carboxylate unit are adjacent to each other, and thus, the hydrophilic polymer has high biocompatibility, and is used suitably.

Examples of the method of immobilizing the hydrophilic polymer in the hollow fiber membrane include a method in which a hydrophilic solution is applied to the dense surface of the hollow fiber membrane. and then. the hollow fiber membrane and the hydrophilic polymer are cross-linked by irradiation, heat treatment, use of a cross-linking agent, or the like to immobilize the hydrophilic polymer. The immobilization may also be achieved by cross-linking reaction caused by performing irradiation or heat treatment in a state where the hollow fiber membrane is in contact with the hydrophilic solution. A method of producing a hollow fiber membrane in the present invention is not particularly limited, and is preferably a producing method comprising a step of irradiating the membrane with a radiation in a state where the hydrophilic solution is kept in contact with the hollow fiber membrane (the step is referred to as Step 1).

The term "hydrophilic solution" means a solution containing a hydrophilic polymer.

In general, the radiation dose in Step 1 is preferably 5 kGy or more and 50 kGy or less.

In place of Step 1, cross-linking reaction by heat treatment may be performed, and in this case, the heating condition is suitably 120°C or more and 300°C or less.

The step of immobilizing the hydrophilic polymer in the hollow fiber membrane may be performed before incorporating the hollow fiber membrane into a module case, or may be performed after incorporating the hollow fiber membrane into the module case, or after fixing the hollow fiber membrane in the module case. Among these, a method in which cross-linking and immobilization are performed by irradiation with a radiation before incorporating the hollow fiber membrane into the module case, in a state where the hydrophilic solution is in contact with the hollow fiber membrane, i.e.. a producing method including Step 1 is suitable because this method is less prone to allow heat and a cross-linking agent to modify the module material other than the hydrophilic solution and the hollow fiber membrane.

In the present invention. examples of the radiation to be used in Step 1 include $\alpha$ rays, $\beta$ rays, $\gamma$ rays, X rays, ultraviolet rays, and electron rays. In addition, a blood-contacting material needs to be sterilized. An irradiation sterilization method in which $\gamma$ rays or electron rays are used is often used. That is, performing cross-linking using irradiation makes it possible to perform sterilization simultaneously with immobilizing the hydrophilic polymer on the hollow fiber membrane. In cases where the sterilization and the cross-linking are performed simultaneously, the irradiation dose is preferably 15 kGy or more. On the other hand, the irradiation dose at 50 kGy or more raises concern that the hydrophilic polymer may be cross-linked or decomposed, or that a module material other than the hollow fiber membrane or the hollow fiber membrane may deteriorate. Thus, the irradiation dose is more preferably 15 kGy or more and 50 kGy or less.

In addition, using an antioxidant during irradiation with a radiation makes it possible to control the cross-linking reaction in the hydrophilic polymer, and to prevent the modification of a polyolefin that is a material for the hollow fiber membrane.

The term "antioxidant" means a molecule having a property by which an electron is easy to donate to another molecule. Examples thereof include: watersoluble vitamins. such as vitamin C; polyphenols; alcohols, such as ethanol, propanol. ethylene glycol, propylene glycol, and glycerin; sugars, such as glucose galactose, mannose, and trehalose; inorganic salts, such as sodium hydrosulfite, sodium pyrosulfite, and sodium dithionate; uric acid; cysteine; and glutathione. These antioxidants may be used individually, or may be used in combination of two or more kinds thereof. In cases where the antioxidant is used as a blood-contacting material for medical use, an antioxidant that is suitably used as an antioxidant having low biological toxicity is ethanol, n-propanol, 2-propanol, ethylene glycol, propylene glycol, or glycerin.

The amount of the antioxidant to be added to the hydrophilic solution varies depending on the kind of the antioxidant. In particular, in the case of ethanol, *n*-propanol, or 2-propanol, the amount is preferably 0.01 mass% or more and 30.0 mass% or less. more preferably 0.05 mass% or more and 20.0 mass% or less, from the viewpoint of cross-linking the hollow fiber membrane and the hydrophilic polymer suitably, and from the viewpoint of preventing the materials and the like from being deteriorated by a radiation.

In many of the cases where the equilibrium constant of adsorption of the hydrophilic polymer to the hollow fiber membrane is high, the amount of adsorption into the hollow fiber membrane is large, causing the hydrophilization of the hollow fiber membrane to progress, and decreasing the plasma-leak resistance. In this case, decreasing the concentration of the hydrophilic polymer contained in the hydrophilic solution, or decreasing the amount of the hydrophilic solution for the hollow fiber membrane makes it possible to adjust the amount of the hydrophilic polymer to be immobilized on the surface of the hollow fiber membrane. In addition, it is possible to adjust the thickness of the hydrophilic polymer, using an antioxidant having polarity. For example, in cases where the antioxidant is ethanol. the polarity of ethanol can increase the affinity of the hydrophilic polymer for a hydrophobic base material. Accordingly, adjusting the ethanol concentration of the hydrophilic polymer solution enables the thickness of the hydrophilic polymer to be adjusted.

In cases where the hollow fiber membrane is PMP, and where the hydrophilic polymer is a copolymer of vinylpyrrolidone and vinyl propionate. a copolymer of polyvinylpyrrolidone and vinyl acetate, a copolymer of polyvinylpyrrolidone and vinyl butanoate, a copolymer of polyvinylpyrrolidone and vinyl pivalate. a copolymer of polyvinylpyrrolidone and vinyl nonanoate, a copolymer of polyvinylpyrrolidone and vinyl decanoate, a copolymer of N-isopylacrylamide unit and ethyl acrylate unit, a copolymer of vinylacetamide unit and vinyl pivalate unit, or polyvinylpyrrolidone, the hydrophilic polymer concentration of the hydrophilic solution is preferably 10 ppm or more and 5000 ppm or less, more preferably 100 ppm or more and 5000 ppm or less, from the viewpoint of maintaining the hydrophobicity of the hollow fiber membrane to suppress plasma leak, or from the viewpoint of suppressing the decrease in the gas permeability by the cross-linking of the hydrophilic polymer itself. In addition, the ethanol concentration is preferably 0.01 mass% or more and 20.0 mass% or less, more preferably 0.1 mass% or more and 20.0 mass% or less.

For a method of producing the hollow fiber membrane according to the present invention, it is preferable that, in Step 1, only one surface of the hollow fiber membrane, *i.e.,* the dense surface that is a blood-contacting face, is irradiated with a radiation in a state where the hydrophilic solution is kept in contact with the surface. Examples of the method of bringing the hydrophilic solution into contact with the outer surface of the hollow fiber membrane include a method in which the ends of the hollow fiber membrane are embedded in a resin or the like. and the cross-linking and the immobilization are achieved by irradiating the surface with a radiation in a state where the hydrophilic solution is prevented from exuding into the inside of the hollow fiber membrane. Then, those portions of the hollow fiber membrane which are embedded in the resin are cut off to afford a hollow fiber membrane having a hydrophilic polymer cross-linked and immobilized on the outer surface thereof.

In addition, in cases where the hydrophilic polymer is cross-linked and immobilized in a state where the hollow fiber membrane is incorporated in a module case, the hydrophilic solution can be held only in the space in contact with the outer surface of the hollow fiber membrane because a retaining plate or retaining resin of the module case, at the ends of the hollow fiber membrane, can partition off the respective spaces on the outer surface and inner surface of the hollow fiber membrane. In this state, the outer surface can be irradiated with a radiation to afford a hollow fiber membrane module containing a hollow fiber membrane having a hydrophilic polymer cross-linked and immobilized in the outer surface.

In addition, examples of a method of bringing the hydrophilic solution into contact with only the inner surface include a method in which the hydrophilic solution is fed into the inside of the hollow fiber membrane, which is then irradiated with a radiation in a sealed state to cross-link the hydrophilic polymer. When the hydrophilic solution is fed into the inside, the liquid is preferably fed under pressure, and thus, is preferably fed in a state where the hollow fiber membranes have been inserted into, and held in, the module case. Examples of a sealant for the ends of the hollow fiber membrane include urethane resins, epoxy resins, and acryl resins. From the viewpoint of resistance to, and biocompatibility with, irradiation, urethane resins are preferable. In addition, a module case into which the hollow fiber membranes are suitably used to be inserted is for example, polystyrene or polycarbonate from the viewpoint of radiation resistance.

The hollow fiber membrane module according to the present invention has a hollow fiber membrane according to the present invention packed therein. A method of packing hollow fiber membranes into a hollow fiber membrane module according to the present invention is not particularly limited, and a hollow fiber membrane bundle in the form of a bundle. layer, coil, or woven fabric may be packed.

A hollow fiber membrane module according to the present invention is suitable for a hollow fiber membrane oxygenator that removes carbonic acid gas, such as carbon dioxide, from blood, and adds oxygen to the blood, particularly in extracorporeal blood circulation. In cases where the hollow fiber membrane is used for a hollow fiber membrane oxygenator, the hollow fiber membrane is used in combination with a circuit for removing blood from, and transfusing blood into, the body, and besides, a catheter, pump, blood reserver, temperature regulator, gas line, gas cylinder, gas regulator, biomonitor, and device monitor.

The hollow fiber membrane according to the present invention preferably has a nitrogen gas permeability of 0.02 mL/[min·cm$^2$ bar] or more. The hollow fiber membrane having a nitrogen gas permeability of 0.02 mL/[min·cm$^2$ bar] or more has a sufficient gas-permeating performance when used as an oxygenator. From the viewpoint of enhancing the gas exchange performance of the oxygenator, the nitrogen gas permeability of the hollow fiber membrane is more preferably 0.20 mL/[min·cm$^2$·bar] or more, still more preferably 0.30 mL/[min·cm$^2$·bar] or more. In this regard, the upper limit of the nitrogen gas permeability of the hollow fiber membrane is not particularly set. and is preferably 50.00 mL/[min·cm$^2$·bar] or less from the viewpoint of the proneness to cause plasma leak.

Examples

The present invention will be described below with reference to Examples and Comparative Examples. The present invention is not limited to these Examples.

<Thickness of hydrophilic-polymer-containing layer>

By way of example, an embodiment in which a hydrophilic polymer containing a nitrogen atom is used will be described. Also in cases where a hydrophilic polymer containing no nitrogen atom is used, a measurement can be made in the same manner by detecting a peak derived from an atom or chemical structure unique to the hydrophilic polymer.

A hollow fiber membrane having an outer surface in the form of a dense surface, in which outer surface a hydrophilic polymer containing a nitrogen atom was immobilized, was dried. While the outer surface of the hollow fiber membrane was etched in the thickness direction of the membrane, the intensity of the peak detected for the secondary ion derived from the hydrophilic polymer and the intensity of the peak detected for the secondary ion derived from a main component of the hollow fiber membrane were measured using GCIB-TOF-SIMS. The thickness of the hydrophilic-polymer-containing layer was the distance over which the hydrophilic-polymer-containing layer was etched in the thickness direction of the membrane until a value obtained by dividing the sum of the intensities of the peaks for the secondary ions $CNO^-$ and $C_4HNO^-$ derived from the hydrophilic polymer, by the intensity of the peak for the $C_2H^-$ ion derived from PMP as a main component of the hollow fiber membrane became 0.5.

More specifically, the distance over which the membrane was etched in the thickness direction thereof, using TOF-SIMS5 (manufactured by ION-TOF GmbH), was regarded as the hydrophilic-polymer-containing layer, wherein the membrane was etched until a value obtained by dividing the sum of the intensities of the peaks for the negative secondary ions $CNO^-$ (m/z = 42.00.) and $C_4HNO^-$ (m/z = 84.04) detected as the negative secondary ions derived from the hydrophilic polymer, by the intensity of the peak for the negative secondary ion of the linear hydrocarbon $C_2H^-$ (m/z = 25.01) of PMP of the hollow fiber membrane became 0.5.

In this regard, the intensity of the secondary-ion peak was the intensity of the average secondary-ion peak of a 200 $\mu$m square, and was measured each time the membrane was etched. In addition, the etching depth was each etching depth (6.16 nm) that was a value obtained by dividing the etching depth (733 nm) at the position of measurement by the total number of times of etching (120 times), in which the etching depth was measured using a stylus surface roughness meter after a GCIB-TOF-SIMS measurement. The secondary-ion peak intensity at each measurement interval was calculated by performing linear approximation from the measurement values of two points. one before or after the other. In this regard, the measurement conditions for GCIB-TOF-SIMS were as follows.

Primary ion: $Bi_3^{++}$
Secondary-ion polarity: only negative ion
Accelerating voltage: primary ion. 30 kV
Pulse width: 11.3 ns
Bunching: performed
Range of mass for detection (m/z): 0 to 2500
Size of raster: 200 $\mu$m
Number of scans: 1 scan/cycle
Number of pixels: 128 pixels
Degree of vacuum measured: (before introduction of a sample) $4 \times 10^{-7}$ Pa or less
Antistatic measures: taken
After-acceleration: 9.5 kV
Etching ion: Ar-GCIB
Etching-ion accelerating voltage: 5 kV
Size of Ar cluster: 1500 (median value)

The same measurement was made at two different positions, and the average of the measurements was calculated. Here, a value rounded to one decimal place was used as the thickness of a hydrophilic-polymer-containing layer.

<Hydration energy density of hydrophilic polymer>

The hydration energy of a monomer unit was defined using the molecular model of a monomer unit. In the molecular model of a monomer unit, an object used for calculation was a structure having a repeating unit an end of which was a linear alkane. For quantum-chemistry calculation, Gaussian 09, Revision D.01 (manufactured by Gaussian, Inc.)

was used, and for the Connolly surface, MaterialsStudio (manufactured by BIOVIA) was used.

The hydration energy of the above-described monomer unit was calculated using the following method. First, the monomer unit in vacuum was structurally optimized, and then, the in-vacuo energy and underwater energy of the structurally optimized structure were calculated.

In the structure-optimizing step, the density functional theory was used. As a functional, B3LYP was used, and as a basis function, 6-31G (d,p) was used. Furthermore, opt was set as a keyword to be written in the input file.

Next, the in-vacuo energy and underwater energy of the structurally optimized structure were calculated. The in-vacuo energy was calculated using a density functional. As a functional, B3LYP was used, and as a basis function, 6-31G (d.p) was used. The underwater energy was calculated using the density functional theory. As a functional, B3LYP was used, and as a basis function, 6-31G (d,p) was used. Furthermore. to calculate the underwater energy, the continuum dielectric model was utilized, and as the keywords, the following were used.

SCRF = (PCM, G03Gefaults, Read, Solvent = Water)
Radii = UAHF
Alpha =1.20

The hydration energy of the above-described monomer unit was determined by determining the in-vacuo SCF energy and the underwater SCF energy. Here, the SCF energy means the value of E, which is the value written on the line shown by "SCF Done:". For calculation of the above-described energy, the quantum-chemistry calculation software Gaussian 09, Revision D.01 (manufactured by Gaussian, Inc.) was used. The hydration energy density of the hydrophilic polymer is defined on the basis of the following Formula (1).

[Math. 2]

$$\text{Hydration energy density of copolymer } (\text{kJ·mol}^{-1}\text{·nm}^{-3}) =$$

$$\sum_{i=1}^{N}\{(\text{Molar fraction of monomer unit } i) \times (\text{Hydration energy of monomer unit } i)\} \;/$$

$$\sum_{i=1}^{N}\{(\text{Molar fraction of monomer unit } i) \times (\text{Volume of monomer unit } i)\} \;\ldots$$

Formula (1)

[in Formula (1) above, the hydration energy of the monomer unit i is the absolute value of the value obtained by subtracting the energy of the monomer unit i in vacuum from the energy of the monomer unit i in water; N represents the total number of monomer species constituting the copolymer; and i represents an integer of 1 or more and N or less.]

The volume of the monomer unit was calculated utilizing the Connolly surface method in Materials Studio (manufactured by BIOVIA).

The parameters used for the calculation were as follows.

Grid resolution = Coarse
Grid interval = 0.075 nm
vdW factor = 1.0
Connolly radius = 0.1 nm

The hydration energy density of the hydrophilic polymer was defined in accordance with Formula (1) on the basis of a volume calculated utilizing the hydration energy and the Connolly surface method. For the volume of the monomer unit in the above-described Formula (1), the above-described structurally optimized structure was used. Here, a value rounded off to whole numbers was used as the hydration energy density. In this regard, for the molar fraction in Formula (1), the value obtained by calculation in "Molar fraction of monomer unit" below can be used. In cases where the molar fraction cannot be calculated through $^{1}$H-NMR measurement, for example, because the peaks overlap each other. the molar fraction may be calculated through elemental analysis.

<Molar fraction of monomer unit>

A hydrophilic polymer in an amount of 2 mg was dissolved in 2 mL of 99.7% chloroform-D (having a 0.05 volume% TMS, manufactured by Fujifilm Wako Pure Chemical Corporation). The resulting solution was introduced into a sample tube for $^{1}$H-NMR measurement, and a $^{1}$H-NMR measurement (superconductive FITNMREX-270, manufactured by JEOL) was performed. The temperature was set to room temperature, and the number of scans to accumulate was set to 32. Using the measurement results, the value of $A_{PVP}/(A_{PVP}+A_{VC})$ was calculated and used as the molar fraction of a vinylpyrrolidone unit, wherein $A_{PVP}$ is the area of the region surrounded by the baseline and the peak observed at 2.7 ppm or more and 4.3 ppm or less, and derived from the proton ($^3$H) bound to a carbon atom adjacent to the nitrogen atom of vinylpyrrolidone, and wherein $A_{VC}$ is the area of the region surrounded by the baseline and the peak observed at 4.3 ppm or more and 5.2 ppm or less, and derived from the proton ($^1$H) bound to the $\alpha$-position carbon of vinyl carboxylate. In this regard, the molar fraction was calculated as a value rounded to one decimal place.

<Nitrogen atom content>

The nitrogen atom content of a 100 mass% hollow fiber membrane was measured as follows: a hollow fiber membrane having a nitrogen-containing hydrophilic polymer immobilized therein was dried and cut so as to have a given mass; and the amount of nitrogen atoms contained in the membrane was measured using an oxidative decomposition/reduced-pressure chemiluminescence method, using a trace nitrogen analyzer (model ND-100, manufactured by Mitsubishi Chemical Corporation). More specifically, the hollow fiber membrane approximately 15 cm was thermally decomposed and oxidized, and the nitrogen monoxide generated was measured using the chemiluminescence method. The amount was quantitated through calculation from a calibration curve prepared using a standard pyridine solution. The settings for the measurement were as follows.

Electric oven temperature: the portion to be thermally decomposed, 800°C; the catalyst portion, 900°C
Main $O_2$ flow rate: 300 mL/min
Sub $O_2$ flow rate: 300 mL/min
Ar flow rate: 400 mL/min
Sensing mode: High

In this regard, the average of the resultant values obtained by making a measurement three times was used as a measurement value, as which a value rounded to four decimal places was used.

<Vinylpyrrolidone unit content>

The vinylpyrrolidone unit content of a 100 mass% hollow fiber membrane was determined by converting the nitrogen atom content to the vinylpyrrolidone unit content in accordance with the following Formula (2) because one nitrogen atom (the atomic weight: 14) is contained in the vinylpyrrolidone unit (the molecular weight: 111), wherein the nitrogen atom content was calculated in "Nitrogen atom content" above.

Vinylpyrrolidone unit content (mass%) = nitrogen content (mass%) $\times$ (molecular weight of vinylpyrrolidone unit/atomic weight of nitrogen)                Formula (2)

Here, a value rounded to three decimal places was used as the vinylpyrrolidone content.

<Static contact angle of water>

The static contact angle of water on the dense surface of the hollow fiber membrane was measured by the sessile drop method, using an automatic microscopic contact angle meter (MCA-4, Kyowa Interface Science Co.. Ltd.). As the liquid for measurement, distilled water was used, and a measurement was made under conditions at room temperature and a humidity of $30\pm2$%. The amount of the liquid droplets was $44.7\pm3.4$ pL. The droplets were discharged at 115 kPa $\times$18 ms onto the outer surface of the hollow fiber membrane through a capillary having a diameter of 5 $\mu$m, wherein the outer surface was a dense surface. The liquid droplets were measured 250 times at 10 ms intervals, and analyzed by the $\theta$/2 method. In addition, the first image wherein water droplets were attached to the sample was used for the analysis. A measurement was repeated three times, and the average value was calculated. Here, a value rounded to one decimal place was used as the static contact angle of water.

<Nitrogen gas permeability>

As the gas permeability of the hollow fiber membrane, the permeation gas volume was determined by measuring a per-unit-time pressure variation of the nitrogen gas on the permeated side, using the external pressure method at a measurement temperature of 37°C in accordance with the pressure sensor method in JIS K7126-1 (2006). The measurement conditions were as follows.

Supply pressure of nitrogen gas: 0.04 MPa (gauge pressure)
Volume on the permeated side: 475.9 cm$^3$
Measurement time: 4 minutes

For the measurement, a module containing hollow fiber membranes 7 cm in length, and sealed and fixed with an epoxy resin was used. The gas permeability was determined in accordance with the following Formula (3). The average value of the results obtained by making a measurement three times was calculated.

Nitrogen gas permeability (mL/[min·cm$^2$·bar]) = permeation gas volume (mL)/ [time (min) × outer surface area (cm$^2$) of hollow fiber membrane × pressure difference (bar)]          Formula (3)

Here, a value rounded to two decimal places was used as the nitrogen gas permeability.

<Amount of adhesion of protein>

A hollow fiber membrane having a length of 1.5 m, and having both ends sealed with an epoxy resin was washed using phosphate buffered saline (hereinafter referred to as "PBS"). Next, the hollow fiber membrane was immersed in 10 mL of a solution of 80 g/L albumin in PBS, and incubated at 37°C for 72 hours. After the immersion, the hollow fiber membrane was washed with PBS, then immobilized with a 2.5% glutaraldehyde solution in physiological saline, and dried. Next, the hollow fiber membrane was immersed in 1.8 mL of the Working reagent of a BCA assay kit (Micro BCA Protein Assay Kit. product number: 23235, manufactured by Thermo Fisher Scientific Inc.), and incubated at 60°C for 60 minutes. The absorbance of the Working reagent after the reaction was measured at 562 nm, and the amount of adhesion of the protein remaining in the hollow fiber membrane was calculated. Here, a value rounded to one decimal place was used as the amount of adhesion of protein.

<Number of platelets adhering>

Hollow fiber membranes having the outer surface exposed were attached in a row to the bottom face of a sample cup, and brought into contact with fresh human blood. Then, the hollow fiber membranes washed and fixed were observed using a scanning electron microscope (hereinafter referred to as an "SEM"). More specifically, as shown below. fresh human blood was collected with 10% ACD-A, and centrifuged at 160 G for 25 minutes to afford platelet rich plasma containing a large number of platelets (hereinafter referred to as "PRP"). The hollow fiber membranes immobilized on the bottom face of the sample cup were preliminarily washed with PBS.

After 1 mL of PRP was aliquoted in the sample cup, adenosine-2-phosphate as a platelet-activating stimulant was added to a concentration of 100 μmol/L. The resulting mixture was shaken for 15 minutes. After 15 minutes elapsed, the PRP in the sample cup was discarded, and 1 mL of physiological saline was added. Shaking and disposal were repeated approximately 10 times, and platelets were removed from the sample cup by washing. Subsequently, a 2.5% glutaraldehyde solution in physiological saline was added to the sample cup, and left to stand for 1 hour or more. The platelets adhering to the hollow fiber membranes were fixed.

After the fixing liquid was discarded, the fixed sample was washed with water for injection, and dried using a vacuum dryer (FD-1, manufactured by Tokyo Rikakikai Co., Ltd.) for 1 hour or more. For observation with an SEM, a Pt sputter (E-1045, manufactured by Hitachi High-Tech Fielding Corporation) was used under conditions at a discharge current of 15 mA and for a time of 40 seconds. Using an SEM (SEMEDX Type-HS-3000, manufactured by Hitachi High-Tech Fielding Corporation), the platelets adhering to the outer surfaces of the hollow fiber membranes were observed. The observation was made at a magnification of 1500 times and at a viewing angle of $4.35 \times 10^4$ μm$^2$. The average value of 20 fields of view was used as the number of platelets adhering.

In this regard, the count per field of view was up to 50, and more than 50 was treated as >50. The average was determined, and a value rounded off to whole numbers was used as the number of platelets adhering. In addition, in cases where a plurality of platelets spread and deformed, thus being unable to be counted one by one. and where the adhesion of platelets was observed in the majority of a field of view. the result was treated as thrombus formation.

<State of adhesion of platelets>

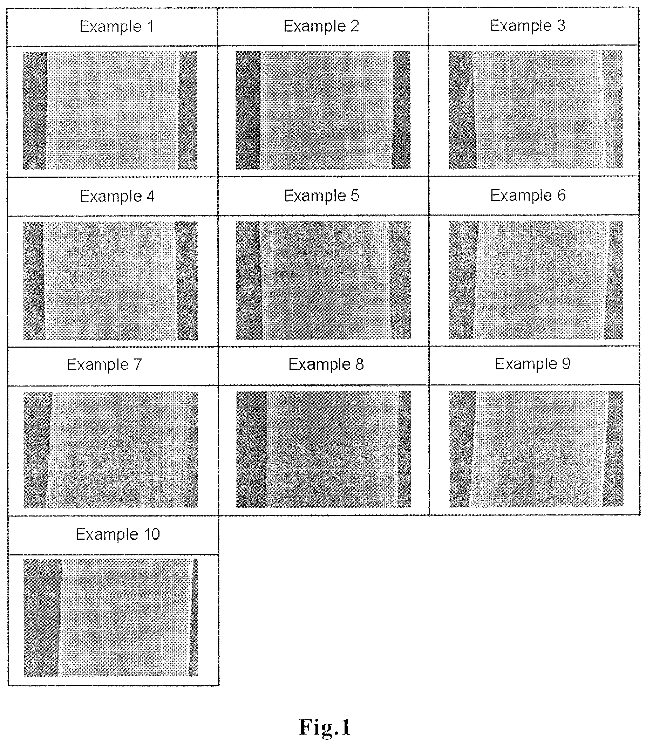

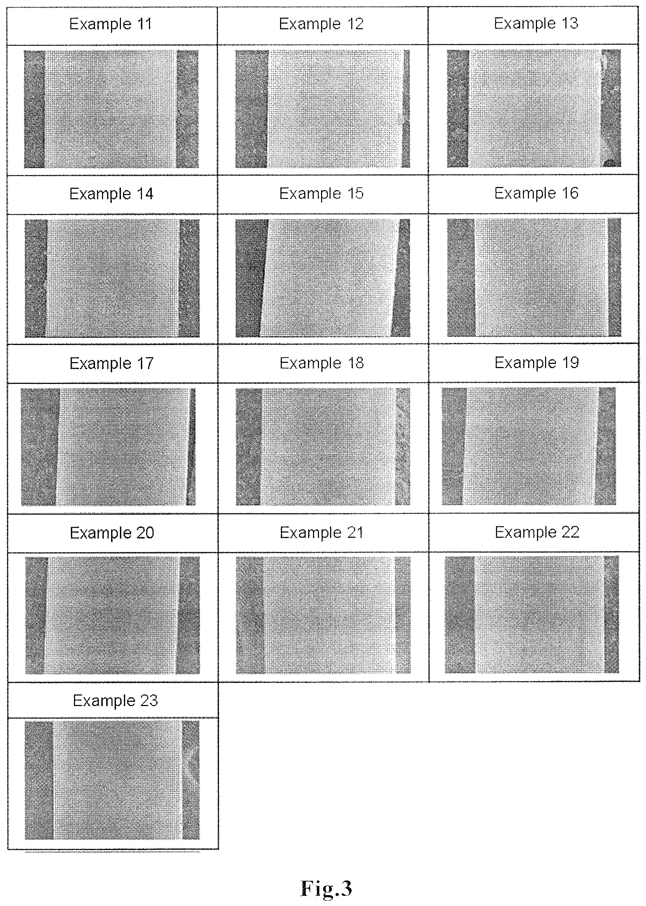

Hollow fiber membranes produced and sputtered in the same manner as in the measurement of the number of platelets adhering were used to observe the thrombi adhering to the hollow fiber membranes. The observation was made using an SEM (SEMEDX Type-HS-3000, manufactured by Hitachi High-Tech Fielding Corporation) at a magnification of 250 times. As shown in FIG. 1 and FIG. 3. almost no adhesion of platelets was observed in Examples, but as shown in FIG.

2, the adhesion of platelets was observed in Comparative Examples. In this regard, in cases where thrombi were formed on the whole of the hollow fiber membranes. the result was treated as thrombus formation.

[Example 1]

The hollow fiber membrane used was composed of PMP, had an outer diameter of 380 $\mu$m, an inner diameter of 200 $\mu$m, and a thickness of 90 $\mu$m, and had the outer surface in the form of a dense surface. The ends of the hollow fiber membrane were sealed so that only the outer surface could come into contact with a liquid. This hollow fiber membrane was immersed in a hydrophilic solution containing a random copolymer of vinylpyrrolidone units and vinyl propionate units (of which copolymer, the weight-average molecular weight was 58,000. the molar fraction of the vinylpyrrolidone units was 0.6, and the hydration energy density was 182 kJ·mol$^{-1}$·nm$^{-3}$) at 10 ppm and ethanol at 0.1 mass%, and irradiated with $\gamma$ ray at 25 kGy. The hollow fiber membrane irradiated with $\gamma$ ray was washed with PBS to obtain a hollow fiber membrane 1.

[Example 2]

A hollow fiber membrane 2 was obtained by performing the same operation as in Example 1 except that the concentration of the random copolymer of vinylpyrrolidone units and vinyl propionate units in the hydrophilic solution was changed to 100 ppm.

[Example 3]

A hollow fiber membrane 3 was obtained by performing the same operation as in Example 1 except that the concentration of the random copolymer of vinylpyrrolidone units and vinyl propionate units in the hydrophilic solution was changed to 200 ppm.

[Example 4]

A hollow fiber membrane 4 was obtained by performing the same operation as in Example 1 except that the concentration of the random copolymer of vinylpyrrolidone units and vinyl propionate units in the hydrophilic solution was changed to 500 ppm, and that the ethanol concentration was changed to 1.0 mass%.

[Example 5]

A hollow fiber membrane 5 was obtained by performing the same operation as in Example 4 except that the ethanol concentration of the hydrophilic solution was changed to 10.0 mass%.

[Example 6]

A hollow fiber membrane 6 was obtained by performing the same operation as in Example 4 except that the ethanol concentration of the hydrophilic solution was changed to 20.0 mass%.

[Example 7]

A hollow fiber membrane 7 was obtained by performing the same operation as in Example 1 except that the concentration of the random copolymer of vinylpyrrolidone units and vinyl propionate units in the hydrophilic solution was changed to 1000 ppm, and that the ethanol concentration was changed to 10.0 mass%.

[Example 8]

A hollow fiber membrane 8 was obtained by performing the same operation as in Example 7 except that the ethanol concentration of the hydrophilic solution was changed to 20.0 mass%.

[Example 9]

A hollow fiber membrane 9 was obtained by performing the same operation as in Example 1 except that the concentration of the random copolymer of vinylpyrrolidone units and vinyl propionate units in the hydrophilic solution was changed to 5000 ppm, and that the ethanol concentration was changed to 10.0 mass%.

[Example 10]

A hollow fiber membrane 10 was obtained by performing the same operation as in Example 9 except that the ethanol concentration of the hydrophilic solution was changed to 20.0 mass%.

[Comparative Example 1]

A hollow fiber membrane 11 was obtained by performing the same operation as in Example 1 except that the ethanol concentration of the hydrophilic solution was changed to 10.0 mass%.

[Comparative Example 2]

A hollow fiber membrane 12 was obtained by performing the same operation as in Example 1 except that the concentration of the random copolymer of vinylpyrrolidone units and vinyl propionate units in the hydrophilic solution was changed to 1 ppm, and that the ethanol concentration was changed to 0.1 mass%.

[Comparative Example 3]

A hollow fiber membrane 13 was obtained by performing the same operation as in Example 1 except that the concentration of the random copolymer of vinylpyrrolidone units and vinyl propionate units in the hydrophilic solution was changed to 15,000 ppm, and that the ethanol concentration was changed to 20.0 mass%.

With respect to the hollow fiber membranes 1 to 13 obtained, Table 1 shows the results of measurements of the thickness of the hydrophilic-polymer-containing layer, the hydration energy density of the hydrophilic polymer, the kind of the hydrophilic unit, the kind of the hydrophobic unit, the nitrogen atom content of the hollow fiber membrane, the vinylpyrrolidone unit content, the static contact angle of water on the dense surface. and the nitrogen gas permeability. and Table 2 shows the results of measurements of the amount of adhesion of protein and the number of platelets adhering.

[Table 1]

| | Thickness of hydrophilic-polymer-containing layer [nm] | Hydration energy density of hydrophilic polymer [kJ·mol⁻¹·nm⁻³] | Kind of hydrophilic unit | Kind of hydrophobic unit | Nitrogen atom content [mass%] | Hydrophilic unit content [mass%] | Static contact angle of water [°] | Nitrogen gas permeability [mL/[min·cm²·bar] |
|---|---|---|---|---|---|---|---|---|
| Example 1 | 6.3 | 182 | vinylpyrrolidone | vinyl propionate | 0.0024 | 0.020 | 86.3 | 0.49 |
| Example 2 | 4.7 | 182 | vinylpyrrolidone | vinyl propionate | 0.0018 | 0.015 | 88.0 | 0.48 |
| Example 3 | 25.0 | 182 | vinylpyrrolidone | vinyl propionate | 0.0094 | 0.079 | 80.1 | 0.20 |
| Example 4 | 5.5 | 182 | vinylpyrrolidone | vinyl propionate | 0.0021 | 0.017 | 89.0 | 0.43 |
| Example 5 | 7.6 | 182 | vinylpyrrolidone | vinyl propionate | 0.0036 | 0.030 | 87.2 | 0.49 |
| Example 6 | 3.5 | 182 | vinylpyrrolidone | vinyl propionate | 0.0017 | 0.014 | 90.0 | 0.59 |
| Example 7 | 8.0 | 182 | vinylpyrrolidone | vinyl propionate | 0.0037 | 0.031 | 84.5 | 0.40 |
| Example 8 | 4.5 | 182 | vinylpyrrolidone | vinyl propionate | 0.0021 | 0.018 | 88.4 | 0.48 |
| Example 9 | 11.2 | 182 | vinylpyrrolidone | vinyl propionate | 0.0053 | 0.044 | 82.1 | 0.32 |
| Example 10 | 12.5 | 182 | vinylpyrrolidone | vinyl propionate | 0.0059 | 0.049 | 81.6 | 0.30 |
| Comparative Example 1 | - | - | - | - | - | - | 99.8 | 0.56 |
| Comparative Example 2 | 0.9 | 182 | vinylpyrrolidone | vinyl propionate | 0.0004 | 0.003 | 97.0 | 0.50 |
| Comparative Example 3 | 41.6 | 182 | vinylpyrrolidone | vinyl propionate | 0.0178 | 0.149 | 75.6 | 0.01 |

[Table 2]

| | Amount of adhesion of protein [μg/m²] | Number of platelets adhering [platelets/4.35×10⁴ μm²] |
|---|---|---|
| Example 1 | 23.4 | 36 |
| Example 2 | 24.9 | 49 |
| Example 3 | 19.4 | 38 |
| Example 4 | 22.6 | 47 |
| Example 5 | 21.1 | 26 |
| Example 6 | 26.4 | 8 |
| Example 7 | 19.7 | 19 |
| Example 8 | 25.5 | 16 |
| Example 9 | 20.1 | 11 |
| Example 10 | 20.0 | 9 |
| Comparative Example 1 | 30.9 | thrombus formation |
| Comparative Example 2 | 27.0 | >50 |
| Comparative Example 3 | 20.5 | 36 |

[Example 11]

A hollow fiber membrane 14 was obtained by performing the same operation as in Example 1 except that the hydrophilic solution was changed so as to have polyvinylpyrrolidone (the weight-average molecular weight: 40,000; the molar fraction of the vinylpyrrolidone units: 1.0; and the hydration energy density: 213 kJ·mol$^{-1}$·nm$^{-3}$) at a concentration of 200 ppm and ethanol at 0.1 mass%.

[Example 12]

A hollow fiber membrane 15 was obtained by performing the same operation as in Example 11 except that the polyvinylpyrrolidone concentration of the hydrophilic solution was changed to 1000 ppm, and that the ethanol concentration was changed to 20 mass%.

[Example 13]

A hollow fiber membrane 16 was obtained by performing the same operation as in Example 1 except that the hydrophilic solution was changed so as to have the random copolymer of vinylpyrrolidone units and vinyl acetate units (of which copolymer, the weight-average molecular weight was 58,000, the molar fraction of the vinylpyrrolidone units was 0.6, and the hydration energy density was 187 kJ·mol$^{-1}$·nm$^{-3}$) at a concentration of 200 ppm and ethanol at 0.1 mass%.

[Example 14]

A hollow fiber membrane 17 was obtained by performing the same operation as in Example 13 except that the hydrophilic solution was changed so as to have the random copolymer of vinylpyrrolidone units and vinyl acetate units at a concentration of 1000 ppm and ethanol at 10 mass%.

[Example 15]

A hollow fiber membrane 18 was obtained by performing the same operation as in Example 13 except that the hydrophilic solution was changed so as to have the random copolymer of vinylpyrrolidone units and vinyl acetate units at a concentration of 7500 ppm and ethanol at 0.1 mass%.

[Example 16]

A hollow fiber membrane 19 was obtained by performing the same operation as in Example 1 except that the hydrophilic solution was changed so as to have the random copolymer of vinylpyrrolidone units and vinyl butanoate units (of which copolymer, the weight-average molecular weight was 21,000, the molar fraction of the vinylpyrrolidone units was 0.7, and the hydration energy density was 193 $kJ \cdot mol^{-1} \cdot nm^{-3}$ at a concentration of 500 ppm and ethanol at 20 mass%.

[Example 17]

A hollow fiber membrane 20 was obtained by performing the same operation as in Example 16 except that the hydrophilic solution was changed so as to have the random copolymer of vinylpyrrolidone units and vinyl butanoate units at a concentration of 1000 ppm and ethanol at 20 mass%.

[Example 18]

A hollow fiber membrane 21 was obtained by performing the same operation as in Example 1 except that the hydrophilic solution was changed so as to have the random copolymer of vinylpyrrolidone units and vinyl pivalate units (of which copolymer, the weight-average molecular weight was 39,000, the molar fraction of the vinylpyrrolidone units was 0.7, and the hydration energy density was 172 $kJ \cdot mol^{-1} \cdot nm^{-3}$) at a concentration of 200 ppm and ethanol at 10 mass%.

[Example 19]

A hollow fiber membrane 22 was obtained by performing the same operation as in Example 18 except that the hydrophilic solution was changed so as to have a random copolymer of vinylpyrrolidone units and vinyl pivalate units at a concentration of 500 ppm and ethanol at 20 mass%.

[Example 20]

A hollow fiber membrane 23 was obtained by performing the same operation as in Example 1 except that the hydrophilic solution was changed so as to have a random copolymer of vinylpyrrolidone units and vinyl nonanoate units (of which copolymer, the weight-average molecular weight was 44,000, the molar fraction of the vinylpyrrolidone units was 0.8, and the hydration energy density was 167 $kJ \cdot mol^{-1} \cdot nm^{-3}$) at a concentration of 500 ppm and ethanol at 10 mass%.

[Example 21]

A hollow fiber membrane 24 was obtained by performing the same operation as in Example 1 except that the hydrophilic solution was changed so as to have a random copolymer of vinylpyrrolidone units and vinyl decanoate units (of which copolymer, the weight-average molecular weight was 19,000, the molar fraction of the vinylpyrrolidone units was 0.8, and the hydration energy density was 171 $kJ\text{-}mol^{-1} \cdot nm^{-3}$) at a concentration of 500 ppm and ethanol at 10 mass%.

[Example 22]

A hollow fiber membrane 25 was obtained by performing the same operation as in Example 1 except that the hydrophilic solution was changed so as to have a random copolymer of N-isopylacrylamide units and ethyl acrylate unit (of which copolymer, the weight-average molecular weight was 3000. the molar fraction of the N-isopylacrylamide was 0.5, and the hydration energy density was 181 $kJ \cdot mol^{-1} \cdot nm^{-3}$) at a concentration of 500 ppm and ethanol at 15 mass%.

[Example 23]

A hollow fiber membrane 26 was obtained by performing the same operation as in Example 1 except that the hydrophilic solution was changed so as to have a random copolymer of vinylacetamide units and vinyl pivalate units (of which copolymer, the weight-average molecular weight was 76,000, the molar fraction of the vinylacetamide units was 0.5, and the hydration energy density was 178 kJ $\cdot$mol$^{-1}\cdot$nm$^{-3}$) at a concentration of 500 ppm and ethanol at 20 mass%.

[Comparative Example 4]

A hollow fiber membrane 27 was obtained by performing the same operation as in Example 11 except that the polyvinylpyrrolidone concentration of the hydrophilic solution was changed to 20 ppm, and that the ethanol concentration was changed to 0.1 mass%.

[Comparative Example 5]

A hollow fiber membrane 28 was obtained by performing the same operation as in Example 13 except that the hydrophilic solution was changed so as to have a random copolymer of vinylpyrrolidone units and vinyl acetate units at a concentration of 20 ppm and ethanol at 0.1 mass%.

[Comparative Example 6]

A hollow fiber membrane 29 was obtained by performing the same operation as in Example 18 except that the hydrophilic solution was changed so as to have a random copolymer of vinylpyrrolidone units and vinyl pivalate units at a concentration of 50 ppm and ethanol at 20 mass%.

[Comparative Example 7]

A hollow fiber membrane 30 was obtained by performing the same operation as in Example 1 except that the hydrophilic solution was changed so as to have polyvinyl alcohol (the weight-average molecular weight: 95,000; and the hydration energy density: 1531 at a concentration of 1000 ppm and ethanol at 0.1 mass%.

With respect to the hollow fiber membranes 14 to 30 obtained, Tables 3 and 4 show the results of measurements of the thickness of the hydrophilic-polymer-containing layer, the hydration energy density of the hydrophilic polymer, the kind of the hydrophilic unit, the kind of the hydrophobic unit, the nitrogen atom content of the hollow fiber membrane, the vinylpyrrolidone unit content, and the static contact angle of water on the dense surface, and Table 5 shows the results of measurements of the nitrogen gas permeability, the amount of adhesion of protein, and the number of platelets adhering.

[Table 3]

| | Thickness of hydrophilic-polymer-containing layer [nm] | Hydration energy density of hydrophilic polymer [kJ·mol⁻¹·nm⁻³] | Kind of hydrophilic unit | Kind of hydrophobic unit | Nitrogen atom content [mass%] | Hydrophilic unit content [mass%] | Static contact angle of water [°] | Nitrogen gas permeability [mL/(min·cm²·bar)] |
|---|---|---|---|---|---|---|---|---|
| Example 11 | 11.0 | 213 | vinylpyrrolidone | — | 0.0039 | 0.0310 | 90.3 | 0.49 |
| Example 12 | 7.6 | 213 | vinylpyrrolidone | — | 0.0013 | 0.0103 | 89.1 | 0.67 |
| Example 13 | 4.4 | 187 | vinylpyrrolidone | vinyl acetate | 0.0023 | 0.0277 | 88.6 | 0.40 |
| Example 14 | 22.7 | 187 | vinylpyrrolidone | vinyl acetate | 0.0039 | 0.0470 | 88.2 | 0.41 |
| Example 15 | 14.5 | 187 | vinylpyrrolidone | vinyl acetate | 0.0025 | 0.0301 | 74.0 | 0.49 |
| Example 16 | 24.6 | 193 | vinylpyrrolidone | vinyl butanoate | 0.0091 | 0.0618 | 82.7 | 0.20 |
| Example 17 | 23.4 | 193 | vinylpyrrolidone | vinyl butanoate | 0.0089 | 0.0605 | 84.5 | 0.26 |
| Example 18 | 12.8 | 172 | vinylpyrrolidone | vinyl pivalate | 0.0077 | 0.0523 | 90.2 | 0.27 |
| Example 19 | 23.9 | 172 | vinylpyrrolidone | vinyl pivalate | 0.0093 | 0.6320 | 90.9 | 0.21 |
| Example 20 | 14.7 | 167 | vinylpyrrolidone | vinyl nonanoate | 0.0048 | 0.0381 | 86.6 | 0.26 |
| Example 21 | 12.2 | 171 | vinylpyrrolidone | vinyl decanoate | 0.0052 | 0.0381 | 89.9 | 0.36 |
| Example 22 | 10.3 | 181 | N-isopylacrylamide | ethyl acrylate | 0.0062 | 0.0500 | 82.0 | 0.26 |
| Example 23 | 4.4 | 178 | vinylacetamide | vinyl pivalate | 0.0040 | 0.0255 | 84.8 | 0.27 |

[Table 4]

| | Thickness of hydrophilic-polymer-containing layer [nm] | Hydration energy density of hydrophilic polymer [kJ·mol⁻¹·nm⁻³] | Kind of hydrophilic unit | Kind of hydrophobic unit | Nitrogen atom content [mass%] | Hydrophilic unit content [mass%] | Static contact angle of water [°] | Nitrogen gas permeability [mL/[min·cm²·bar] |
|---|---|---|---|---|---|---|---|---|
| Comparative Example 4 | 1.1 | 213 | vinylpyrrolidone | — | 0.0005 | 0.0037 | 96.3 | 0.27 |
| Comparative Example 5 | 1.6 | 187 | vinylpyrrolidone | vinyl acetate | 0.0020 | 0.0241 | 95.5 | 0.20 |
| Comparative Example 6 | 4.9 | 172 | vinylpyrrolidone | vinyl pivalate | 0.0008 | 0.0054 | 98.7 | 0.31 |
| Comparative Example 7 | 1.1 | 153 | vinyl alcohol | — | — | — | 72.4 | 0.18 |

[Table 5]

| | Amount of adhesion of protein [$\mu g/m^2$] | Number of platelets adhering [platelets/$4.35\times10^4\ \mu m^2$] |
|---|---|---|
| Example 11 | 22.3 | 49 |
| Example 12 | 22.8 | 48 |
| Example 13 | 21.0 | 46 |
| Example 14 | 20.9 | 41 |
| Example 15 | 21.3 | 30 |
| Example 16 | 22.6 | 42 |
| Example 17 | 23.1 | 31 |
| Example 18 | 19.4 | 32 |
| Example 19 | 19.9 | 27 |
| Example 20 | 21.1 | 30 |
| Example 21 | 20.8 | 28 |
| Example 22 | 19.8 | 9 |
| Example 23 | 19.2 | 10 |
| Comparative Example 4 | 39.6 | thrombus formation |
| Comparative Example 5 | 40.5 | thrombus formation |
| Comparative Example 6 | 30.4 | >50 |
| Comparative Example 7 | 42.9 | thrombus formation |

The hollow fiber membranes 1 to 10 and 14 to 26 presented the amount of adhesion of protein and the number of platelets adhering that were both small, no thrombus formation on the whole of each hollow fiber membrane, and a gas permeability of 0.2 mL/[min·cm$^2$·bar] or more, and thus, were membranes having both biocompatibility and gas permeability. This is considered to be because the thickness of the layer containing a hydrophilic polymer was sufficient to suppress the adhesion of an organismic component, and was a thickness that did not cause a decrease in the gas permeability. In addition, the static contact angle of water was 70° or more, and thus, the membranes are considered to have such hydrophobicity as to suppress plasma leak.

On the other hand, in the hollow fiber membranes 11, 12, and 27 to 30, the thickness of the hydrophilic-polymer-containing layer was insufficient, and thus, the amount of adhesion of protein and the number of platelets adhering were large. Furthermore, the hollow fiber membranes 11 and 27 to 30 underwent thrombus formation on the whole of each hollow fiber membrane, and were membranes having low biocompatibility. In the hollow fiber membrane 13. the thickness of the layer containing a hydrophilic polymer was large, and thus, the membrane had low gas permeability. Furthermore, the static contact angle of water was 80° or less, which means strong hydrophilic, and thus, the membrane is considered to be prone to cause plasma leak. In the hollow fiber membrane 30, the hydration energy density of the hydrophilic polymer was small, and thus, the membrane is considered to be prone to undergo adhesion of protein and platelets, and cause plasma leak.

Industrial Applicability

A blood component is taken as an example of the contacting liquid in the present invention, but the liquid is not limited to a blood component. The hollow fiber membrane can be used for, for example: cells; microorganisms; somatic cells obtained by treating cultured cells or biological tissues of plants; germ cells; somatic stem cells; cell suspensions composed of induced pluripotent stem cells or embryonic hepatocytes; blood preparations, such as platelet preparations and red cell preparations; and dispersion liquids of microparticles, such as synthetic microparticles, inorganic microparticles, or vesicles. Furthermore, the hollow fiber membrane can be used for: rainwater, seawater, or earth-and-sand liquid in the environment; a synthetic polymer solution; or the like. The hollow fiber membrane can also be utilized as an oxygenating membrane or a degassing membrane for these solutions.

**Claims**

1. A hollow fiber membrane comprising polymethylpentene as a main component, and having a dense surface having a hydrophilic polymer immobilized thereon,
   wherein the thickness of a layer containing said hydrophilic polymer is 1.0 nm or more and 25.0 nm or less.

2. The hollow fiber membrane according to claim 1, wherein the hydration energy density of said hydrophilic polymer is 167 kJ·mol$^{-1}$·nm$^{-3}$ or more and 213 kJ·mol$^{-1}$·nm$^{-3}$ or less.

3. The hollow fiber membrane according to claim 1 or 2, comprising a nitrogen atom in an amount of 0.0010 mass% or more and 0.0100 mass% or less.

4. The hollow fiber membrane according to any one of claims 1 to 3, wherein said hydrophilic polymer comprises a hydrophilic unit selected from the group consisting of a vinylpyrrolidone unit, acrylamide derivative unit, methacrylamide derivative unit, N-vinylacetamide derivative unit, and vinylcaprolactam unit, wherein the amount of said hydrophilic unit is 0.010 mass% or more and 0.080 mass% or less.

5. The hollow fiber membrane according to any one of claims 1 to 4, wherein the contact angle of water on said dense surface is 70° or more and 95° or less.

6. The hollow fiber membrane according to any one of claims 1 to 5, wherein said hydrophilic polymer comprises a hydrophobic unit selected from the group consisting of a vinyl carboxylate unit, alkyl acrylate unit, and alkyl methacrylate unit.

7. The hollow fiber membrane according to claim 6, wherein the number of carbon atoms of a side-chain end of said hydrophobic unit is 1 or more and 9 or less.

8. A method of producing the hollow fiber membrane according to any one of claims 1 to 7, comprising the following Step 1:
   Step 1: a step of irradiating, with a radiation, said hollow fiber membrane brought into contact with a hydrophilic solution.

9. The method of producing a hollow fiber membrane according to claim 8, wherein said hydrophilic solution is brought into contact with only one surface of said hollow fiber membrane.

10. A hollow fiber membrane module in which said hollow fiber membrane according to any one of claims 1 to 7 is packed.

11. The hollow jiber membrane module according to claim 10, which is an oxygenator.

| Example 1 | Example 2 | Example 3 |
|---|---|---|
| | | |
| Example 4 | Example 5 | Example 6 |
| | | |
| Example 7 | Example 8 | Example 9 |
| | | |
| Example 10 | | |
| | | |

**Fig.1**

| Comparative Example 1 | Comparative Example 2 | Comparative Example 3 |
|---|---|---|
| | | |
| Comparative Example 4 | Comparative Example 5 | Comparative Example 6 |
| | | |
| Comparative Example 7 | | |
| | | |

Fig.2

| Example 11 | Example 12 | Example 13 |
|---|---|---|
| | | |
| Example 14 | Example 15 | Example 16 |
| | | |
| Example 17 | Example 18 | Example 19 |
| | | |
| Example 20 | Example 21 | Example 22 |
| | | |
| Example 23 | | |
| | | |

Fig.3

## INTERNATIONAL SEARCH REPORT

| International application No. |
| --- |
| **PCT/JP2024/007213** |

**A.    CLASSIFICATION OF SUBJECT MATTER**

*B01D 71/26*(2006.01)i; *A61M 1/18*(2006.01)i; *B01D 63/02*(2006.01)i; *B01D 69/00*(2006.01)i; *B01D 69/02*(2006.01)i; *B01D 69/08*(2006.01)i; *B01D 69/12*(2006.01)i; *B01D 71/76*(2006.01)i

FI:   B01D71/26; B01D63/02; B01D69/00; B01D69/02; B01D69/08; B01D69/12; B01D71/76; A61M1/18 500; A61M1/18 525

According to International Patent Classification (IPC) or to both national classification and IPC

**B.    FIELDS SEARCHED**

Minimum documentation searched (classification system followed by classification symbols)

B01D53/22; B01D61/00-71/82; C02F1/44; A61M1/14-1/32

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2021/182608 A1 (TORAY INDUSTRIES, INC.) 16 September 2021 (2021-09-16) claims 1-10, paragraphs [0012], [0048], [0112]-[0119], [0128]-[0201] | 1-11 |
| Y | JP 2005-58906 A (ASAHI KASEI MEDICAL CO., LTD.) 10 March 2005 (2005-03-10) claims 1-8, paragraph [0017] | 1-11 |
| Y | JP 2019-130522 A (TORAY INDUSTRIES, INC.) 08 August 2019 (2019-08-08) claims 1-13, paragraph [0043] | 2, 8-9 |
| A | WO 2018/062451 A1 (TORAY INDUSTRIES, INC.) 05 April 2018 (2018-04-05) | 1-11 |
| A | JP 2021-142162 A (TERUMO KABUSHIKI KAISHA) 24 September 2021 (2021-09-24) | 1-11 |
| A | WO 2022/229748 A1 (3M INNOVATIVE PROPERTIES COMPANY) 03 November 2022 (2022-11-03) | 1-11 |

☐ Further documents are listed in the continuation of Box C.       ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 May 2024** | **14 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
**Information on patent family members**

International application No.

**PCT/JP2024/007213**

| Patent document cited in search report | | | Publication date (day/month/year) | Patent family member(s) | | | Publication date (day/month/year) |
|---|---|---|---|---|---|---|---|
| WO | 2021/182608 | A1 | 16 September 2021 | US | 2023/0074745 | A1 | |
| | | | | claims 1-10, paragraphs [0011], [0058], [0122]-[0129], [0138]-[0211] | | | |
| | | | | EP | 4119587 | A1 | |
| | | | | CN | 115175969 | A | |
| JP | 2005-58906 | A | 10 March 2005 | (Family: none) | | | |
| JP | 2019-130522 | A | 08 August 2019 | (Family: none) | | | |
| WO | 2018/062451 | A1 | 05 April 2018 | US | 2021/0197141 | A1 | |
| | | | | EP | 3520886 | A1 | |
| JP | 2021-142162 | A | 24 September 2021 | (Family: none) | | | |
| WO | 2022/229748 | A1 | 03 November 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004357826 A
- JP H11114056 A
- JP 2020141901 A
- JP 2020127705 A
- JP H0316626 A
- JP 2011078974 A

**Non-patent literature cited in the description**

- **M. NIINOMI**. Metals for Biomedical Devices. Elsevier, 2019, 405